# EUROPEAN PATENT SPECIFICATION

(11) **EP 0 404 175 B1**
(45) Date of publication and mention of the grant of the patent: **27.04.1994**
(21) Application number: 90111875.2
(22) Date of filing: 22.06.1990
(51) Int. Cl.: C07D 405/06

(54) **Process for preparing imidazole derivatives**
Verfahren zur Herstellung von Imidazol-Derivaten
Procédé pour la préparation de dérivés d'imidazole

(30) Priority: 22.06.1989 JP 158403/89
(43) Date of publication of application: 27.12.1990
(73) Proprietor: MITSUI PETROCHEMICAL INDUSTRIES, LTD., Tokyo 100 (JP)
(72) Inventor: Ori, Aiichiro, C/o Mitsui Petrochemical Ind., Ltd., Kuga-gun, Yamaguchi-ken (JP); Imuta, Junichi, C/o Mitsui Petrochemical Ind., Ltd, Kuga-gun, Yamaguchi-ken (JP); Kihara, Noriaki C/o Mitsui Petrochemical Ind., Ltd, Kuga-gun, Yamaguchi-ken (JP)
(74) Representative: Hansen, Bernd, Dr. Dipl.-Chem.

(56) References cited:
- TETRAHEDRON, vol. 28, no. 4, February 1972, pages 967-972; J.I. DEGRAW: "An improved synthesis of pilocarpine"
- JOURNAL OF PHARMACEUTICAL SCIENCES, vol. 64, no. 10, October 1975, pages 1700-1701; J.I. DEGRAW et al.: "Synthesis of d-pilocarpine-N-14CH3"

## Description

The present invention relates to an industrially advantageous process for preparing imidazole derivatives of the general formula (II): 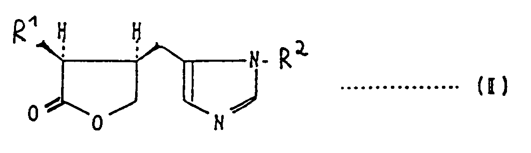
wherein R¹ and R² independently are C₁-C₄ alkyl. These compounds are useful for the treatment of glaucoma.

Both the starting material of the present invention, mercaptoimidazole derivatives (I), and the product imidazole derivatives (II) are well known in the art.

Especially, the compound of the formula (II) wherein R¹ is ethyl and R² is methyl has widely been known under generic name "pilocarpine" as a medicament for the treatment of glaucoma.

The production of imidazole derivatives (II) from mercaptoimidazole derivatives (I) by desulfurization has also been known, for example, using Raney nickel in methoxyethanol [Tetrahedron, 28, 967(1972)] or using hydrogen peroxide [J. Pharmaceutical Sciences, 64, 1700(1975)].

These methods as mentioned above, however, are disadvantageous because of poor yield, the formation of by-product isomer (isopilocarpine), high cost of Raney nickel, and the like.

The object of the present invention is to provide an industrially advantageous process for preparing imidazole derivatives (II) in high yield without the formation of the by-product isomer by desulfurizing the mercaptoimidazole derivative (I), wherein said desulfurization is carried out using inexpensive reagent.

The inventors have surprisingly found that the imidazole derivatives (II) can be obtained in extremely high yield and substantially without the formation of the by-product, by utilizing inexpensive nitric acid and a nitrite as desulfurizing agent.

Thus, the present invention relates to a process for preparing imidazole derivatives of the general formula (II): 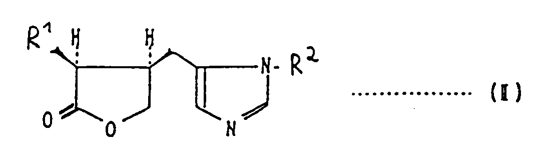
wherein R¹ and R² independently are C₁-C₄ alkyl, which comprises desulfurizing a mercaptoimidazole derivative of the general formula (I): 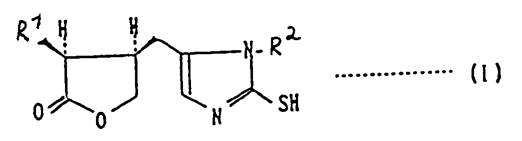
wherein R¹ and R² are as defined above, with 1 to 50 mol per mol of I of nitric acid in the presence of 0.1 to 10 mol of a nitrite.

The mercaptoimidazole derivatives (I) used as a starting material in the present invention are publicly known and can be prepared, for example, according to the method described in Tetrahedron, 28, 967(1972).

The desulfurization of the present invention may be conducted by contacting the starting material (I) with nitric acid in the presence of a nitrite without solvent or in an inert solvent such as water, ethanol, methyl cellosolve, dimethoxyethane, dioxane or acetic acid.

Any nitrites which generate nitrite ion may be used as a nitrite in the process of the present invention.

Typical nitrites which may be mentioned include, for example, inorganic nitrites such as sodium nitrite, potassium nitrite, barium nitrite, etc., and organic nitrites such as dimethylammonium nitrite, dicyclohexylammonium nitrite.

The amount of nitric acid is in the range of 1 to 50 mol, preferably 2 to 20 mol per mol of the starting material mercaptoimidazole derivative (I), and the nitrite is 0.1 to 10 mol, preferably 0.2 to 5 mol. The solvent is used in 2 to 100 times, preferably 5 to 30 times the weight of the starting material.

The reaction is performed at a temperature of -30°C to 100°C, preferably at 3°C to 50°C, for 1 minute to 10 hours, preferably 10 minutes to 5 hours. After the reaction is complete, the imidazole derivative (II) thus formed is isolated after conventional separation and purification.

The following Examples further illustrate the present invention.

Though the compound of the formula (I) wherein R¹ is ethyl and R² is methyl is used as a starting material in the Examples, the present invention is in no way construed as being limited to such compound only. The present invention includes any compounds wherein R¹ and R² individually are C₁-C₄ alkyl such as methyl, ethyl, propyl, butyl.

### Example 1

0.10 g(0.40 mmol) of 3-ethyl-dihydro-4-[(1-methyl-1H-2-mercaptoimidazol-5-yl)methyl]-2(3H)-furanone and 0.3 ml(4.7 mmol) of concentrated nitric acid were added to 2.0 ml of water, and the mixture was stirred for 5 minutes under ice-cooling. To the mixture was added 42 mg(0.60 mmol) of sodium nitrite and the stirring was continued for additional 30 minutes. Then, the temperature of the mixture was raised to room temperature followed by stirring for further 2 hours. After the reaction was complete, the reaction mixture was made alkaline by adding 0.5 ml of 35% aqueous ammonia and extracted with 5 ml of chloroform. The chloroform extract was dried over anhydrous sodium sulfate and evaporated to give 69.3 mg(84%) of pilocarpine as white crystals. The crystals were dissolved in ethanol, filtrated to remove insolubles, 0.1 ml of concentrated hydrochloric acid added, and evaporated to give 77 mg(79%) of white crystals having a m.p. of 200-203°C. ¹H-NMR and mass spectrum (molecular ion peak at 208) of the crystals thus obtained are identical with those of the authentic sample pilocarpine hydrochloride.

### Example 2

The procedure of Example 1 was followed using 7 mg(0.10 mmol) of sodium nitrite instead of 42 mg(0.60 mmol).
Yield of pilocarpine: 85%.

### Example 3

The procedure of Example 1 was followed using 14 mg(0.20 mmol) of sodium nitrite instead of 42 mg(0.60 mmol).
Yield of pilocarpine: 92%.

### Example 4

The procedure of Example 1 was followed using 14 mg(0.20 mmol) of sodium nitrite instead of 42 mg(0.60 mmol), and the reaction was performed at 40°C.
Yield of pilocarpine: 90%.

### Example 5

The procedure of Example 1 was followed using 2.0 ml of acetic acid as a solvent instead of 2.0 ml of water without the presence of a nitrite.
Yield of pilocarpine: 77%.

## Claims

1. A process for preparing imidazole derivatives of the general formula (II): 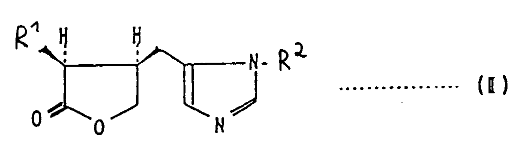 wherein R¹ and R² independently are C₁-C₄ alkyl, which comprises desulfurizing a mercaptoimidazole derivative of the general formula (I): 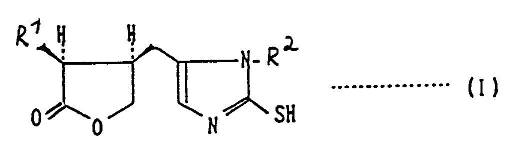 wherein R¹ and R² are as defined above, with 1 to 50 mol per mol of I of nitric acid in the presence of 0.1 to 10 mol of a nitrite.

## Patentansprüche

1. Verfahren zum Herstellen von Imidazolderivaten der allgemeinen Formel (II) 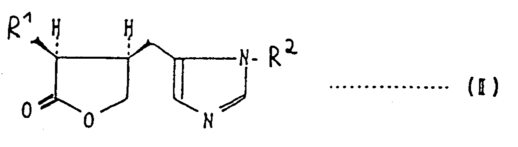 wobei R¹ und R² unabhängig voneinander C₁-C₄-Alkyl sind, welches Desulfurieren eines Mercaptoimidazolderivats der allgemeinen Formel (I) 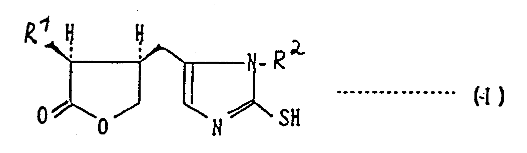 wobei R¹ und R² wie zuvor angegeben definiert sind, mit 1 bis 50 Mol Salpetersäure pro Mol I in Anwesenheit von 0,1 bis 10 Mol eines Nitrits umfaßt.

## Revendications

1. Procédé de préparation de dérivés d'imidazole de formule générale (II) : 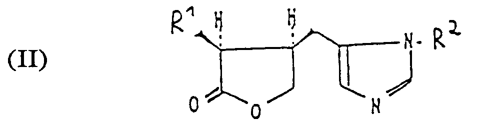 dans laquelle R¹ et R² représentent indépendamment des groupes alkyle en C₁₋₄,
qui comporte la désulfuration d'un dérivé de mercaptoimidazole de formule générale (I) : 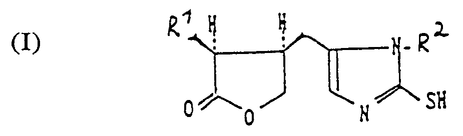 dans laquelle R¹ et R² sont tels que définis ci-dessus,
à l'aide de 1 à 50 moles d'acide nitrique par mole de composé (I), et en présence de 0,1 à 10 moles d'un nitrite.
